(19) **Europäisches Patentamt / European Patent Office / Office européen des brevets**

(11) **EP 2 268 792 B1**

(12) **FASCICULE DE BREVET EUROPEEN**

(45) Date de publication et mention de la délivrance du brevet:
**13.05.2015 Bulletin 2015/20**

(21) Numéro de dépôt: **09728680.1**

(22) Date de dépôt: **18.03.2009**

(51) Int Cl.:
*C12N 1/20* [(2006.01)]     *A23L 1/30* [(2006.01)]
*C12R 1/225* [(2006.01)]     *A61K 35/74* [(2015.01)]

(86) Numéro de dépôt international:
**PCT/FR2009/000284**

(87) Numéro de publication internationale:
**WO 2009/122042 (08.10.2009 Gazette 2009/41)**

(54) **SOUCHE DE LACTOBACILLUS RHAMNOSUS**

LACTOBACILLUS RHAMNOSUS STAMM

STRAIN OF LACTOBACILLUS RHAMNOSUS

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **19.03.2008 FR 0801507**

(43) Date de publication de la demande:
**05.01.2011 Bulletin 2011/01**

(73) Titulaire: **Compagnie Gervais Danone**
**75009 Paris (FR)**

(72) Inventeurs:
• **CHAMBAUD, Isabelle**
  **F-92130 Issy les Moulineaux (FR)**
• **KHLEBNIKOV, Artem**
  **New York 10804 (FR)**
• **VILLAIN, Anne-Catherine**
  **F-92160 Juvisy sur Orge (FR)**
• **GROMPONE, Gianfranco**
  **F-75005 Paris (FR)**
• **SAINT DENIS, Thierry**
  **F-94300 Vincennes (FR)**

(74) Mandataire: **Marcadé, Véronique et al**
**Cabinet Orès**
**36, rue de St Pétersbourg**
**75008 Paris (FR)**

(56) Documents cités:
**WO-A-99/10476**

• **GOPAL P K ET AL:** "In vitro adherence properties of Lactobacillus rhamnosus DR20 and Bifidobacterium lactis DR10 strains and their antagonistic activity against an enterotoxigenic Escherichia coli" INTERNATIONAL JOURNAL OF FOOD MICROBIOLOGY, ELSEVIER SCIENCE PUBLISHERS, AMSTERDAM, NL, vol. 67, no. 3, 5 août 2001 (2001-08-05), pages 207-216, XP002336307 ISSN: 0168-1605
• **FORESTIER CHRISTIANE ET AL:** "Probiotic activities of Lactobacillus casei rhamnosus: In vitro adherence to intestinal cells and antimicrobial properties" RESEARCH IN MICROBIOLOGY, ELSEVIER, AMSTERDAM, NL, vol. 152, no. 2, 1 mars 2001 (2001-03-01), pages 167-173, XP002197762 ISSN: 0923-2508
• **GILL HARSHARNJIT S ET AL:** "Protection against translocating Salmonella typhimurium infection in mice by feeding the immuno-enhancing probiotic Lactobacillus rhamnosus strain HN001." MEDICAL MICROBIOLOGY AND IMMUNOLOGY, vol. 190, no. 3, décembre 2001 (2001-12), pages 97-104, XP002502562 ISSN: 0300-8584
• **COUDEYRAS SOPHIE ET AL:** "Taxonomic and strain-specific identification of the probiotic strain Lactobacillus rhamnosus 35 within the Lactobacillus casei group" APPLIED AND ENVIRONMENTAL MICROBIOLOGY, vol. 74, no. 9, 7 mars 2008 (2008-03-07), pages 2679-2689, XP002502563 ISSN: 0099-2240

EP 2 268 792 B1

- ADLERBERTH I ET AL: "A Mannose-Specific Adherence Mechanism in Lactobacillus plantarum Conferring Binding to the Human Colonic Cell Line HT-29" APPLIED AND ENVIRONMENTAL MICROBIOLOGY, AMERICAN SOCIETY FOR MICROBIOLOGY, US, vol. 62, no. 7, 1 juillet 1996 (1996-07-01), pages 2244-2251, XP002979206 ISSN: 0099-2240

## Description

L'invention est relative à une nouvelle souche de *Lactobacillus rhamnosus* dotée de propriétés antimicrobiennes et immunomodulatrices.

De très nombreuses études scientifiques ont rapporté les effets bénéfiques sur la santé de certains microorganismes présents dans des aliments fermentés, notamment des produits laitiers. Ces microorganismes sont communément dénommés «probiotiques». Selon la définition généralement admise à l'heure actuelle, les probiotiques sont : « des microorganismes vivants, qui lorsqu'ils sont consommés en quantités adéquates, ont un effet bénéfique sur la, santé de l'hôte » (Rapport FAO/OMS sur l'évaluation des propriétés sanitaires et nutritionnelles des probiotiques dans les aliments, y compris le lait en poudre contenant des bactéries lactiques vivants ; Cordoba, Argentine ; 1-4 octobre 2001).

Il a été montré que la consommation de produits alimentaires contenant des bactéries probiotiques peut produire des effets favorables sur la santé, par l'intermédiaire notamment du rééquilibrage de la flore intestinale, de l'amélioration de la résistance aux infections, et de la modulation de la réponse immunitaire.

Les microorganismes probiotiques utilisés en alimentation humaine sont généralement des bactéries lactiques, appartenant principalement aux genres *Lactobacillus* et *Bifidobacterium.*

Les effets bénéfiques sur la santé ne sont toutefois généralement pas communs à l'ensemble des bactéries d'un même genre, ni même d'une même espèce. Ils ne se rencontrent, le plus souvent, que chez certaines souches ; en outre, les effets observés peuvent varier qualitativement et/ou quantitativement d'une souche probiotique à l'autre, y compris à l'intérieur d'une même espèce.

Pour qu'un microorganisme puisse être considéré comme étant potentiellement utilisable comme probiotique, il doit satisfaire à au moins un, et idéalement plusieurs, des critères suivants :

- présenter une activité inhibitrice vis-à-vis de microorganismes pathogènes pouvant être présents dans la flore intestinale, cette activité pouvant résulter soit de la capacité à adhérer aux cellules intestinales, excluant ou réduisant ainsi l'adhérence des pathogènes, soit de la capacité à produire des substances inhibant les pathogènes, soit de la combinaison de ces deux caractéristiques
- présenter des propriétés immunomodulatrices, et notamment immunostimulantes et/ou anti-inflammatoires.

En outre, si ce microorganisme est destiné à être incorporé dans un produit laitier, il doit de préférence présenter une croissance satisfaisante sur lait.

Enfin, il doit conserver une bonne viabilité, au cours de la production et de la conservation de l'aliment dans lequel il est incorporé, ainsi qu'après l'ingestion de cet aliment par le consommateur, de manière à pouvoir atteindre l'intestin et à survivre dans le milieu intestinal.

Il est toutefois à noter que bien que la viabilité soit essentielle pour répondre à la définition actuelle de « probiotique », il a été montré que certains des effets bénéfiques associés à des souches probiotiques pouvaient être obtenus même en l'absence de bactéries vivantes, et étaient attribuables à certaines fractions bactériennes ou à des fractions actives de leurs surnageants de culture. Par exemple, la Demande PCT WO2004093898 décrit une préparation immunomodulatrice obtenue par fractionnement du surnageant de culture dé la souche CNCM I-2219.

Les Inventeurs sont maintenant parvenus à isoler une souche de *Lactobacillus rhamnosus* répondant aux critères indiqués ci-dessus.

La présente invention a pour objet cette souche, qui a été déposée selon le Traité de Budapest, auprès de la CNCM (Collection Nationale de Cultures de Microorganismes, 25 rue du Docteur Roux, Paris), le 9 novembre 2006, sous le numéro I-3690.

Elle possède les caractéristiques suivantes:

- Morphologie : Petits bacilles trapus parfois coccoides, isolés ou petites chaines.
- Fermentation des sucres suivants (résultats obtenus sur une galerie api 50 CH - Milieu MRS API à 37°C pendant 48h) : Ribose, galactose, D-glucose, D-fructose, D-mannose, L-sorbose, rhamnose, mannitol, sorbitol, Methyl-D glucoside, N-acétylglucosamine,arbutine, esculine, salicine, maltose, lactose, trehalose, melezitose, beta gentiobiose, D-turanose, D-tagatose, gluconate.

Elle possède d'autre part des propriétés anti-microbiennes, se traduisant par une forte capacité à inhiber la croissance en culture de microorganismes pathogènes.

Elle présente en outre des propriétés d'adhésion au mannose. Il est connu que des glycoconjugués riches en mannose présents à la surface des cellules épithéliales intestinales jouent un rôle dans l'attachement de bactéries pathogènes, telles que les *Escherichia coli* entérotoxiques, les salmonelles, *Vibrio cholerae* ou *Pseudomonas aeruginosa,* et il a été rapporté que les propriétés d'adhésion au mannose de certaines bactéries probiotiques leur permettaient d'entrer en compétition avec ces pathogènes, et d'inhiber leur adhésion à la muqueuse intestinale, leur conférant ainsi des propriétés anti-infectieuses (MICHAIL et ABERNATHY, Pediatr. Gastroenterol. Nutr. , 35, 350-355 2002 ; MANGELL et al., Dig Dis Sci., 47, 511-506, 2002). Ces propriétés avaient toutefois été observées principalement sur des bactéries de l'espèce *Lactobacillus plantarum* (notamment la souche *L. plantarum* 229v, décrite dans la Demande EP0817640 et la souche *L. plantarum* WCFS1), mais pas sur des bactéries de l'espèce *Lactobacillus rhamnosus.*

La souche CNCM I-3690 possède également des propriétés immunomodulatrices, et notamment anti-inflammatoires.

La présente invention a également pour objet un procédé d'obtention d'une souche de *Lactobacillus rhamnosus,* caractérisé en ce qu'il comprend la mutagenèse ou la transformation génétique de la souche CNCM I-3690, ainsi que toute souche de *Lactobacillus rhamnosus* susceptible d'être obtenue par ce procédé, et conservant les propriétés anti-microbiennes et les propriétés immunomodulatrices de la souche CNCM I-3690.Il peut s'agir de souches dans lesquelles un ou plusieurs des gènes endogènes de la souche CNCM I-3690 a (ont) été muté(s), par exemple de manière à modifier certaines de ses propriétés métaboliques (e.g. la capacité de cette souche à métaboliser des sucres, sa résistance au transit intestinal, sa résistance à l'acidité, sa post acidification ou sa production de métabolites). Il peut s'agir également de souches résultant de la transformation génétique de la souche CNCM I-3690 par un ou plusieurs gène(s) d'intérêt, permettant par exemple de conférer à ladite souche des caractéristiques physiologiques supplémentaires, ou d'exprimer des protéines d'intérêt thérapeutique ou vaccinal, que l'on souhaite administrer par l'intermédiaire de ladite souche.

Ces souches peuvent être obtenues à partir de la souche CNCM I-3690 par les techniques classiques de mutagénèse aléatoire ou dirigée et de transformation génétique de lactobacilles, telles que celles décrites par exemple par GURY et al. (Arch Microbiol., 182, 337-45, 2004) ou par VÉLEZ et al. (Appl Environ Microbiol., 73, 3595-3604, 2007), ou par la technique dénommée « genome shuffling » (PATNAIK et al. Nat Biotechnol, 20, 707-12, 2002 ; WANG Y. et al., J Biotechnol., 129, 510-15, 2007).

La présente invention a également pour objet un procédé d'obtention de fractions cellulaires possédant des propriétés immunomodulatrice et antimicrobiennes, caractérisé en ce que lesdites fractions sont obtenues à partir d'une souche de *Lactobacillus rhamnosus* conforme à l'invention. Il s'agit notamment de préparations d'ADN ou de préparations de parois bactériennes obtenues à partir de cultures de ladite souche. Il peut s'agir également de surnageants de culture ou de fractions de ces surnageants.

La présente invention a également pour objet des compositions comprenant une souche de *Lactobacillus rhamnosus* conforme à l'invention.

Ces compositions peuvent être notamment des ferments lactiques, associant une souche de *Lactobacillus rhamnosus* conforme à l'invention avec une ou plusieurs autre (s) souche(s) de bactéries lactiques, probiotique(s) ou non.

Il peut s'agir également de produits alimentaires, et notamment de produits laitiers, ou de produits pharmaceutiques ou cosmétiques comprenant une souche de *Lactobacillus rhamnosus* conforme à l'invention.

Une souche de *Lactobacillus rhamnosus* conforme à l'invention peut ainsi être utilisée comme médicament, notamment comme médicament destiné au traitement d'une infection microbienne.

Lorsque ladite souche est présente sous forme de bactéries vivantes, celles-ci seront de préférence présentes à raison d'au moins $10^5$ ufc par gramme, avantageusement au moins $10^6$ ufc par gramme de produit, plus avantageusement au moins $10^7$ ufc par gramme et encore plus avantageusement au moins $10^8$ ufc par gramme.

La présente invention a aussi pour objet un procédé d'obtention d'une composition possédant des propriétés anti-microbiennes et/ou immunomodulatrices, caractérisé en ce qu'il comprend l'obtention d'une fraction cellulaire par un procédé conforme à l'invention tel que décrit ci-dessus, et l'incorporation de ladite fraction dans ladite composition. De préférence, ladite composition est un produit alimentaire.

La présente invention sera mieux comprise à l'aide du complément de description qui va suivre qui se réfère à des exemples illustrant les propriétés anti-microbiennes, immunomodulatrices et anti-infectieuses de la souche CNCM I-3690.

## EXEMPLE 1 : COMPARAISON DES PROPRIÉTÉS DE LA SOUCHE CNCM I-3690 AVEC CELLES DE SOUCHES PROBIOTIQUES CONNUES

Les propriétés de la souche CNCM I-3690 ont été comparées avec celles de différentes souches de l'art antérieur, connues pour leurs propriétés probiotiques.

La liste de ces souches est donnée dans le Tableau I ci-dessous.

Tableau I

| Genre | Espèce | Dénomination(s) | Numéro de publication de Demandes de Brevet |
|---|---|---|---|
| *Lactobacillus* | *johnsonii* | NCC533=La1= CNCM I-1225 | EP0577903 |

(suite)

| Genre | Espèce | Dénomination(s) | Numéro de publication de Demandes de Brevet |
|---|---|---|---|
| *Lactobacillus* | *acidophilus* | NCFM | WO2004032639 |
| *Lactobacillus* | *acidophilus* | La-5 | |
| *Lactobacillus* | *casei* | Shirota | |
| *Lactobacillus* | *paracasei* | CRL431 = ATCC 55544 | |
| *Lactobacillus* | *rhamnosus* | LGG = ATCC 53103 | US4839281 |
| *Lactobacillus* | *rhamnosus* | HN001 = NM97/09514 | WO9910476 |
| *Lactobacillus* | *plantarum* | ATCC 14917=DSMZ 20174=WCFS1 | |
| *Lactobacillus* | *plantarum* | Probi 299v = DSM 9843 | WO9391823 |
| *Lactobacillus* | *reuteri* | DSMZ 20016 | |
| *Lactobacillus* | *reuteri* | Biogaia SD 2112 : ATCC55730 | WO2004034808 |
| *Bifidobacterium* | *breve* | ATCC 15700 | |
| *Bifidobacterium* | *breve* | BBC50 = CNCM 1-2219 | EP1189517 |
| *Bifidobacterium* | *infantis* | ATCC 15697 | |
| *Bifidobacterium* | *longum* | BB536 | |
| *Bifidobacterium* | *longum* | NCC2705 = CNCM I-2618 | |
| *Bifidobacterium* | *animalis subsp. lactis* | BB 12 = ATCC 27536 | |
| *Bifidobacterium* | *animalis subsp. lactis* | HN019 = NM97/01925 | WO9910476 |

**Matériel et méthodes**

**1- Activité antimicrobienne**

La recherche d'activités anti-microbiennes a été effectuée contre trois bactéries pathogènes cibles : *Escherichia coli* E1392-75-2A, *Salmonella enteritidis* NIZO B1241 et *Listeria monocytogenes* 4B. Les bactéries lactiques ont été mises en culture sur boîtes de Pétri dans différents milieux : LM17 (milieu M17 (TERZAGHI & SANDINE, Appl. Microbiol. 29, 807-813, 1975) additionné de 1% de lactose, milieu Elliker (ELLIKER et al., J. Dairy Sci., 39, 1611-1612, 1956), et milieu TGV (Tryptone-Glucose-Meat Extract).

Les boîtes sont incubées à 37°C jusqu'à apparition de colonies bactériennes. Les cultures de *Bifidobacterium* ont été effectuées sous anaérobiose. Une couche d'agar contenant du milieu BHI (brain-heart infusion) et le pathogène est alors coulée à la surface des boîtes. Les boîtes sont incubées de nouveau à 37°C pendant 2 4h. Les diamètres des zones d'inhibition du pathogène sont ensuite mesurés autour de chaque colonie de bactéries lactiques. Le score 1 correspond à un diamètre entre 1 et 3 mm. Le score 2 correspond à un diamètre entre 4 et 6 mm. Le score 3 correspond à un diamètre supérieur à 6 mm. Chaque expérience a été menée trois fois indépendamment pour chaque souche.

Les scores obtenus sur les pathogènes cibles dans chaque expérience ont été additionnés, pour obtenir, pour chaque bactérie lactique, un score global d'activité anti-microbienne.

Les résultats sont donnés dans le Tableau II ci-après.

Ces résultats montrent que parmi les souches testées, la souche CNCM I-3690 est avec la souche ATCC55544 celle qui possède l'activité anti-microbienne la plus élèvée.

**Tableau II**

| Genre | Espèce | Référence | (1) coli/Elliker | (2) coli/ LM17 | (3) Listeria/El-liker | (4) Liste-ria/TGV | (5) Liste-ria/ LM17 | Salmonella/Elliker | (7) Salmonella/TGV | Score anti-pathogènes (1+2+3+4+5+6+7) |
|---|---|---|---|---|---|---|---|---|---|---|
| *Lactobacillus* | *rhamnosus* | DN 116 010 ou CNCM I-3690 | 2 | 1 | 3 | 2 | 0 | 3 | 3 | 14 |
| *Lactobacillus* | *paracasei* | CRL431=ATCC55544 | 2 | 0 | 3 | 3 | 1 | 3 | 2 | 14 |
| *Lactobacillus* | *rhamnosus* | LGG = ATCC 53103 | 2 | 0 | 1 | 2 | 0 | 3 | 3 | 11 |
| *Lactobacillus* | *plantarum* | ATCC 14917= DSMZ 20174=WCFS1 | 2 | 0 | 1 | 2 | 0 | 3 | 2 | 10 |
| *Lactobacillus* | *casei* | Shirota | 2 | 0 | 3 | 1 | 0 | 1 | 2 | 9 |
| *Lactobacillus* | *rhamnosus* | HN001 = NM97/09514 | 1 | 0 | 1 | 2 | 0 | 3 | 2 | 9 |
| *Lactobacillus* | *johnsonii* | NCC533=La1=I-1225 | 1 | 0 | 1 | 1 | 1 | 2 | 1 | 7 |
| *Lactobacillus* | *plantarum* | Probi 299v = DSM 9843 | 1 | 0 | 1 | 1 | 0 | 1 | 1 | 5 |
| *Lactobacillus* | *acidophilus* | La-5 | 1 | 0 | 1 | 2 | 0 | 0 | 1 | 5 |
| *Lactobacillus* | *acidophilus* | NCFM | 1 | 0 | 1 | 1 | 0 | 0 | 1 | 4 |
| *Bifidobacterium* | *animalis subsp. lactis* | BB12 = ATCC 27536 | 1 | 0 | 0 | 0 | 0 | 0 | 2 | 3 |
| *Lactobacillus* | *reuteri* | Biogaia SD 2112 ATCC55730 | 0 | 0 | 1 | 0 | 0 | 1 | 0 | 2 |
| *Bifidobacferium* | *longum* | BB536 | 0 | 0 | 0 | 0 | 0 | 0 | 0 | 0 |
| *Bitidobacterium* | *animalis subsp. lactis* | HN019 = NM97/01925 | 0 | 0 | 0 | 0 | 0 | 0 | 0 | 0 |
| *Lactobacillus* | *reuteri* | DSMZ 20016 | 0 | 0 | 0 | 0 | 0 | 0 | 0 | 0 |
| *Bifidobacterium* | *infantis* | ATCC 15697 | 0 | 0 | 0 | 0 | 0 | 0 | 0 | 0 |
| *Bifidobacterium* | *breve* | BBC50 = I-2219 | 0 | 0 | 0 | 0 | 0 | 0 | 0 | 0 |
| *Bifidobacterium* | *breve* | ATCC 15700 | 0 | 0 | 0 | 0 | 0 | 0 | 0 | 0 |

(suite)

| Genre | Espèce | Référence | (1) coli/Elliker | (2) coli/ LM17 | (3) Listeria/El-liker | (4) Liste-ria/TGV | (5) Liste-ria/ LM17 | Salmonella/Elliker | (7) Salmonella/TGV | Score anti-pathogènes (1+2+3+4+5+6+7) |
|---|---|---|---|---|---|---|---|---|---|---|
| *Bifidobacterium* | *longum* | NCC2705 = CNCM I-2618 | 0 | 0 | 0 | 0 | 0 | 0 | 0 | 0 |

2 - Immunomodulation

Les propriétés immunomodulatrices des différentes bactéries lactiques ont été évaluées par détection de la modulation induite par ces bactéries sur la réponse inflammatoire de cellules épithéliales de colon (HT-29), en mesurant l'effet de ces bactéries sur l'activation du régulateur transcriptionnel NF-$\kappa$B et la sécrétion de la cytokine pro-inflammatoire IL-8 par les cellules HT-29 en présence d'un mélange de TNF$\alpha$, IL-1$\beta$ et IFN$\gamma$ (Cytomix) simulant les conditions d'une agression inflammatoire.

Culture des bactéries lactiques

La croissance des bactéries lactiques s'effectue dans du milieu MRS (DE MAN et al., J. Appl. Bacteriol. 23, 130-135, 1960), ou MRS complémenté avec L-cystéine (1% final), ou dans du milieu Elliker, selon l'espèce bactérienne testée. Les bactéries ont été inoculées et cultivées en une première pré-culture de 10 mL pendant 16h à 37°C, et re-cultivées le lendemain en une deuxième culture dans 100 mL pendant 16h à 37°C, et récoltées en fin de phase stationnaire.

Culture et préparation des cellules

Les cellules HT29 sont entretenues à 37 °C et 5% de $CO_2$ dans du milieu Dulbecco's Modified Eagle Medium (DMEM) complémenté avec 4,5g/L de D-Glucose, L-Glutamine (1mM final), acides aminés (AA) non essentiels (1% final), Pénicilline/Streptomycine (1% final), Sérum de veau foetal (SVF) (10% final).
Les cellules HT-29 sont ensemencées dans des plaques de 12 puits à raison de 2. $10^5$ cellules par puits dans 2ml de milieu, deux jours avant les expériences d'interaction avec les bactéries et de stimulation par le Cytomix.
Pour la mesure de l'activation du régulateur transcriptionnel NF-$\kappa$B les cellules sont transfectées le lendemain de l'ensemencement, avec le plasmide rapporteur Ig-kB-Luciférase, comme décrit par TIEN et al. (J. Immunol., 176, 1228-37, 2006). Préalablement à la transfection, les cellules sont lavées et le milieu est remplacé par le même milieu sans antibiotiques. La transfection est effectuée en déposant dans chaque puits 100 $\mu$L d'un mélange contenant 75 ng de plasmide pIg-kB-luciférase et 1 $\mu$L de Lipofectamine 2000 dans 99 $\mu$L de milieu OptiPro. Les plaques sont ensuite incubées pendant 24H à 37°C et 5% de $CO_2$.

Interaction avec les bactéries et stimulation par le Cytomix

Les bactéries sont récupérées en fin de phase stationnaire, et lavées deux fois au PBS avant d'être mises en interaction avec les cellules.
Elles sont ensuite incubées pendant 2 heures avec les bactéries, avec une multiplicité d'infection de 100 bactéries par cellule. Après deux heures d'interaction avec les bactéries, les cellules sont incubées pendant 6 heures en présence des bactéries et du Cytomix, à raison de 50ng/mL de TNF$\alpha$, 2,5ng/mL d'IL-1$\beta$ et 7,5ng/mL d'IFN$\gamma$ dans le milieu d'incubation. Toutes les incubations sont effectuées à 37°C, et 5% de $CO_2$.
Afin de déterminer le niveau basal d'activation de NF-$\kappa$B et de sécrétion d'IL-8 en l'absence de bactéries lactiques, la même expérience a été réalisée en effectuant la stimulation par le Cytomix pendant 6 heures, après deux heures d'incubation des cellules en l'absence de bactéries.
A l'issue des 8 heures d'incubation, on récupère 1 mL de milieu pour doser l'IL8 sécrétée. Ce dosage est effectué par ELISA en utilisant le kit « Chimioluminescent ELISAs QuantiGlo Human IL-8 » (R&D Systems).
Pour mesurer l'activation de NF-$\kappa$B, les cellules sont lysées dans 100 microlitres d'un tampon (Tris pH 7,9 25mM, $MgCl_2$ 8 mM, Triton 1 %, Glycérol 15 %, additionné de 1mM DTT. Pour mesurer l'activité luciférase, 10 microlitres de lysat cellulaire sont ajoutés à du tampon de lecture (Tris pH 7,9 25 mM, $MgCl_2$ 8 mM, Triton 1%, Glycérol 15 %), additionné de 1mM DTT, 100mM ATP et 2 mM de Luciférine/$K_2HPO_4$ - pH 7,58. La mesure est effectuée à l'aide d'un luminomètre (BECKMAN COULTER).
Après ce temps total d'interaction de 8 heures, on mesure l'activation de NF-$\kappa$B, et la sécrétion d'IL-8.
Les valeurs d'activation de NF-$\kappa$B et de sécrétion d'IL-8 sont exprimées en pourcentages par rapport au niveau basal observé en l'absence de bactéries lactiques. Pour chaque souche testée, un score moyen d'immunomodulation est calculé en additionnant le pourcentage d'activation de NF-$\kappa$B et le pourcentage de-sécrétion d'IL-8. Ces résultats sont rassemblés dans le tableau III suivant.

Tableau III

| Genre | Espèce | Référence | NFκB-HT29 (8) | IL8-HT29 (9) | Score moyen HT29 IL8/NFκB (8+9)/2 |
|---|---|---|---|---|---|
| *Lactobacillus* | *rhamnosus* | DN 116 010 ou CNCM I-3690 | 34,7 | 49,7 | 42 |
| *Lactobacillus* | *paracasei* | CRL431=ATCC55544 | 58,7 | 101,5 | 80 |
| *Lactobacillus* | *rhamnosus* | LGG = ATCC 53103 | 78,0 | 81,0 | 80 |
| *Lactobacillus* | *plantarum* | ATCC 14917= DSMZ 20174=WCFS1 | 80,7 | 91,9 | 86 |
| *Lactobacillus* | *casei* | Shirota | 94,0 | 99,7 | 97 |
| *Lactobacillus* | *rhamnosus* | HN001 = NM97/09514 | 25,0 | 49,7 | 37 |
| *Lactobacillus* | *johnsonii* | NCC533=La1=I-1225 | 86,9 | 92,7 | 90 |
| *Lactobacillus* | *plantarum* | Probi 299v = DSM 9843 | 93,9 | 107,7 | 101 |
| *Lactobacillus* | *acidophilus* | La-5 | 93,8 | 89,5 | 92 |
| *Lactobacillus* | *acidophilus* | NCFM | 85,8 | 95,0 | 90 |
| *Bifidobacterium* | *animalis subsp. lactis* | BB12=ATCC 27536 | 99,4 | 98,0 | 99 |
| *Lactobacillus* | *reuteri* | Biogaia SD 2112 ATCC55730 | 82,0 | 87,9 | 85 |
| *Bifidobacterium* | *longum* | BB536 | 111,6 | 92,0 | 102 |
| *Bifidobacterium* | *animalis subsp. lactis* | HN019 = NM97/01925 | 107,7 | 101,2 | 104 |
| *Lactobacillus* | *reuteri* | DSMZ 20016 | 71,4 | 83,6 | 78 |
| *Bifidobacterium* | *infantis* | ATCC 15697 | 77,5 | 96,3 | 87 |
| *Bifidobacterium* | *breve* | BBC50 = I-2219 | 97,5 | 103,6 | 101 |
| *Bifidobacterium* | *breve* | ATCC 15700 | 117,7 | 104,6 | 111 |
| *Bifidobacterium* | *longum* | NCC2705 = CNCM I-2618 | 87,8 | 91,4 | 90 |

Ces résultats montrent que la souche CNCM I-3690 inhibe fortement la réponse inflammatoire des cellules épithéliales HT-29. Parmi les autres souches testées, seule la souche HN001 possède des propriétés anti-inflammatoires comparables.

**3- Survie aux stress gastrique et intestinal**

Un modèle *in vitro* reflétant les conditions de stress gastrique et intestinal a été utilisé.

Des cultures de bactéries lactiques sont réalisées dans du lait supplémenté avec de l'extrait de levure. Les cultures sont incubées pendant 24 à 48h, selon les espèces (jusqu'à la phase stationnaire de culture).

**Stress intestinal :** Un jus intestinal artificiel composé de sels biliaires de porc (à 3.3 g/l) et de tampon carbonate NaHCO3 (à 16,5 g/l) est réalisé. Le pH est ajusté à 6,3. 1 ml de ce jus intestinal est ajouté à 100 μl de culture bactérienne. Les cultures sont ensuite incubées 5 heures. Ensuite, les populations bactériennes avant et après le stress sont évaluées sur boîtes.

**Stress gastrique :** Un jus gastrique artificiel est réalisé. Il est composé d'acide lactique (9 g/l), de pepsine (3,5 g/l) et de NaCl (2.2 g/l). Le pH est ajusté à 3,1. 1 ml de ce jus artificiel est ajouté à 100 μl de culture bactérienne. Les cultures sont incubées pendant différent temps : 10 min, 30 min et 60 min. Les populations bactériennes sont évaluées sur boîtes.

Les valeurs sont exprimées de la manière suivants :

$$\text{Stress gastrique} = \text{moyenne} [\log (ufc10min/ufc0) \text{ et} \log (ufc0/ufc0)] *10 + \text{moyenne} [\log (ufc30min/ufc0) \text{ et } \log (ufc10min/ufc0)] *20 + \text{moyenne} [\log (ufc60min/ufc0) \text{ et } \log (ufc30min/ufc0)] *30.$$

$$\text{Stress intestinal} = \log (ufc5h/ufc0h).$$

ufcXmin étant la concentration en bactéries exprimée en Unités Formant Colonies (UFC) après X minutes d'incubation.

Pour le stress gastrique, la survie est bonne quand la valeur est supérieure à -50, moyennement bonne quand la valeur est comprise entre -50 et -100 et mauvaise quand la valeur est inférieure à -100.

Pour le stress intestinal, la survie est bonne quand la valeur est supérieure à -0,5, moyennement bonne quand la valeur est comprise entre -0,5 et -1,5 et mauvaise quand la valeur est inférieure à -1,5.

Les résultats sont donnés dans le tableau IV suivant.

Tableau IV

| Genre | Espèce | Référence | Stress intestinal | Stress gastrique |
|---|---|---|---|---|
| Lactobacillus | rhamnosus | DN 116 010 ou CNCM I-3690 | 0,31 | -31,9 |
| Lactobacillus | paracasei | CRL431=ATCC55544 | 0,04 | -47,4 |
| Lactobacillus | rhamnosus | LGG = ATCC 53103 | -0,07 | -44,0 |
| Lactobacillus | plantarum | ATCC 14917= DSMZ 20174=WCFS1 | -0,79 | -68,5 |
| Lactobacillus | casei | Shirota | -1,50 | -139,2 |
| Lactobacillus | rhamnosus | HN001 = NM97/09514 | 0,00 | -86,3 |
| Lactobacillus | johnsonii | NCC533=La1=I-1225 | 0,69 | 20,3 |
| Lactobacillus | plantarum | Probi 299v = DSM 9843 | -1,04 | -29,7 |
| Lactobacillus | acidophilus | La-5 | -0,23 | 43,3 |
| Lactobacillus | acidophilus | NCFM | -1,45 | -63,0 |
| Bifidobacterium | animalis subsp. lactis | BB12 = ATCC 27536 | 0,07 | -70,0 |
| Lactobacillus | reuteri | Biogaia SD 2112 ATCC55730 | 1,00 | -4,6 |
| Bifidobacterium | longum | BB536 | -1,12 | -438,7 |
| Bifidobacterium | animalis subsp. lactis | HN019 = NM97/01925 | 3,40 | 114,0 |
| Lactobacillus | reuteri | DSMZ 20016 | -1,91 | -80,0 |
| Bifidobacterium | infantis | ATCC 15697 | 2,79 | -412,9 |
| Bifidobacterium | breve | BBC50 = 1-2219 | -0,91 | -333,8 |
| Bifidobacterium | breve | ATCC 15700 | -0,14 | -370,2 |
| Bifidobacterium | longum | NCC2705 = CNCM 1-2618 | -0,22 | -319,2 |

Les résultats illustrés dans les Tableaux II, III et IV ci-dessus montrent que, parmi les différentes souches testées, la souche CNCM I-3690 est la seule à présenter à la fois des propriétés anti-microbiennes et des propriétés anti-inflammatoires importantes, accompagnées en outre de très bonnes propriétés de résistance aux stress gastrique et intestinal.

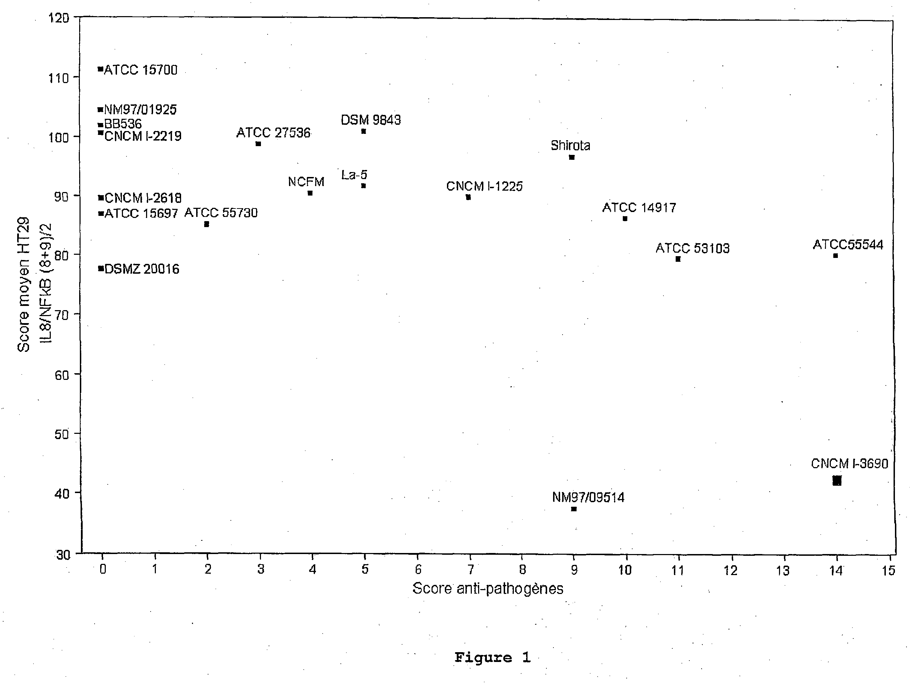

Pour illustrer la supériorité de cette souche, les différents scores obtenus par cette souche sur le plan antimicrobien ont été additionnés entre eux et une moyenne a été faite entre les résultats obtenus par cette souche sur le plan de l'immunomodulation. La Figure 1 montre à quel point cette souche se différencie par rapport aux autres souches testées.

## EXEMPLE 2 : PROPRIETES D'ADHESION AU MANNOSE DE LA SOUCHE CNCM I-3690

La capacité de la souche CNCM I-3690 à adhérer spécifiquement au mannose a été déterminée grâce à un test d'agglutination. Ce test est basé sur la présence de polysaccharides contenant du mannose à la surface des cellules de *Saccharomyces cerevisiae*. Lorsque des bactéries adhérant au mannose sont mises en contact avec *S. cerevisiae,* un phénomène d'agglutination, visible au microscope, intervient.

Ce modèle, permet d'évaluer des propriétés anti-infectieuses potentielles d'une bactérie, résultant de sa capacité à entrer en compétition avec des pathogènes au niveau de l'adhésion à la muqueuse intestinale.

Les souches *L. plantarum* 229v et *L. plantarum* WCFS1, connues pour leur forte capacité d'adhésion au mannose, ont été utilisées à titre de contrôles positifs.

Les souches de *Lactobacillus* sont cultivées en milieu MRS, à une température de 37°C. La croissance des bactéries est arrêtée en phase stationnaire, et les bactéries sont ensuite lavées et ajustées en concentration. La culture de *S. cerevisiae* est effectuée en milieu « malt extract » (Oxoid).

Un volume de 5 μl de suspension bactérienne est ensuite mélangé avec du PBS ou methyl-$\alpha$-D-mannopyranoside (concentration finale : 25 mM). Puis 100 μl d'une préparation à 1 % de cellules de *S. cerevisiae* est ajoutée. Le mélange est agité pendant 10 min à température ambiante, puis examiné au microscope, et des scores sont attribués en utilisant la notation suivante :

Score 0 = pas d'agglutination
Score 1 = faible agglutination
Score 3 = forte agglutination
Score 5 = très forte agglutination.

L'expérience est menée trois fois indépendamment pour chaque souche.

Les résultats sont donnés dans le tableau V ci-après.

Tableau V

| Genre | Espèce | Référence | Score Mannose |
|---|---|---|---|
| *Lactobacillus* | *rhamnosus* | DN 116 010 ou CNCM I-3690 | 3 |
| *Lactobacillus* | *paracasei* | GRL431=ATCC55544 | 0 |
| *Lactobacillus* | *rhamnosus* | LGG = ATCC 53103 | 0 |
| *Lactobacillus* | *plantarum* | ATCC 14917=DSMZ 20174=WCFS1 | 3 |
| *Lactobacillus* | *casei* | Shirota | 0 |
| *Lactobacillus* | *rhamnosus* | HN001 = NM97/09514 | 0 |
| *Lactobacillus* | *johnsonii* | NCC533=La1=I-1225 | 0 |
| *Lactobacillus* | *plantarum* | Probi 299v = DSM 9843 | 3 |
| *Lactobacillus* | *acidophilus* | La-5 | 0 |
| *Lactobacillus* | *acidophilus* | NCFM | 0 |
| *Bifidobacterium* | *animalis subsp. lactis* | BB12 = ATCC 27536 | 0 |
| *Lactobacillus* | *reuteri* | Biogaia SD 2112 ATCC55730 | 0 |
| *Bifidobacterium* | *longum* | BB536 | 0 |
| *Bifidabacterium* | *animalis subsp. lactis* | HN019 = NM97/01925 | 0 |
| *Lactobacillus* | *reuteri* | DSMZ 20016 | 0 |
| *Bifidobacterium* | *infantis* | ATCC 15697 | 0 |
| *Bifidobacterium* | *breve* | BBC50 = 1-2219 | 0 |
| *Bifidobacterium* | *breve* | ATCC 15700 | 0 |
| *Bifidobacterium* | *longum* | NCC2705 = CNCM 1-2618 | 0 |

On observe que le pourcentage de cellules de *S. cerevisiae* agglutinées par la souche CNCM I-3690 est comparable au pourcentage de cellules de *S. cerevisiae* agglutinées par les souches *L. plantarum* 229v et *L. plantarum* WCFS1.

**EXEMPLE 3 : CROISSANCE SUR LAIT DE LA SOUCHE CNCM 1-3690**

Les propriétés de croissance sur lait de la souche CNCM I-3690 ont été testées en utilisant le protocole suivant :

Un milieu constitué de lait écrémé reconstitué par de l'eau additionnée de poudre de lait écrémé a été ensemencé avec la souche CNCM I-3690, à des taux variant entre $5,5\times10^6$ ufc/g et $3,3\times10^7$ ufc/g.

L'activité fermentaire de la souche, reliée à sa croissance, est mesurée en suivant en continu le pH du milieu de croissance.

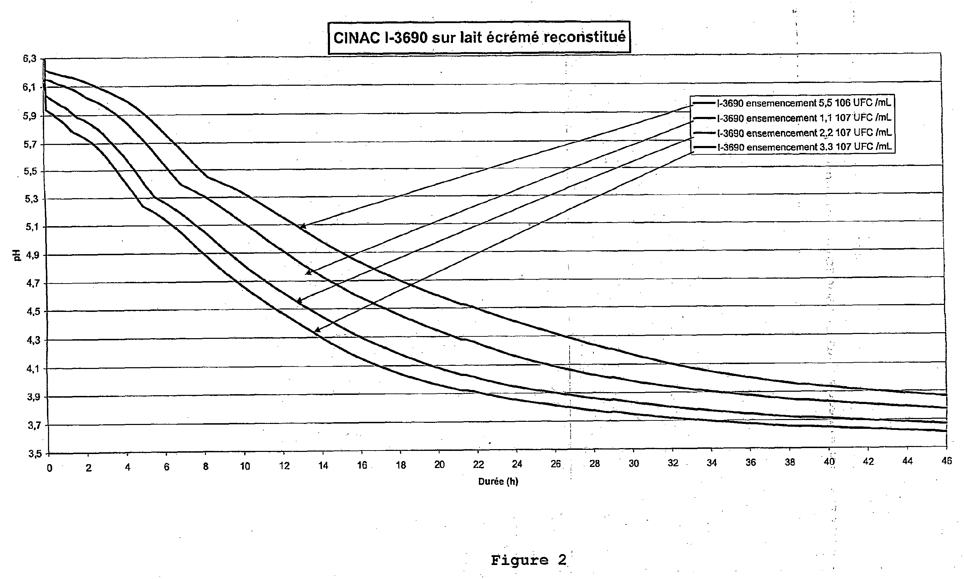

Les résultats sont illustrés par la Figure 2.

Ces résultats montrent que la souche CNCM I-3690 est capable de croître efficacement sur du lait, et qu'elle peut donc être utilisée- dans la fabrication de produits laitiers fermentés.

**Revendications**

1. Souche de *Lactobacillus rhamnosus* possédant des propriétés anti-microbiennes, immunomodulatrices, et d'adhésion spécifique an mannose, déposée auprès de la CNCM sous le numéro I-3690.

2. Procédé d'obtention d'une souche de *Lactobacillus rhamnosus,* **caractérisé en ce qu'**il comprend la mutagenèse ou la transformation génétique de la souche CNCM I-3690.

3. Souche de *Lactobacillus rhamnosus* **caractérisée en ce qu'**elle est susceptible d'être obtenue par le procédé selon la revendication 2, et **en ce qu'**elle possède les propriétés anti-microbiennes et les propriétés immunomodulatrices de la souche CNCM I-3690.

4. Composition comprenant une souche de *Lactobacillus rhamnosus* selon une quelconque des revendications 1 ou 3.

5. Composition selon la revendication 4, **caractérisée en ce qu'**il s'agit d'un produit alimentaire.

6. Souche de *Lactobacillus rhamnosus* selon une quelconque des revendications 1 ou 3 pour l'utilisation comme médicament.

7. Souche de *Lactobacillus rhamnosus* pour l'utilisation comme médicament selon la revendication 6, **caractérisée en ce que** ledit médicament est destiné au traitement d'une infection microbienne.

8. Souche de *Lactobacillus rhamnosus* pour l'utilisation comme médicament selon la revendication 6, **caractérisée en ce que** ledit médicament est anti-inflammatoire.

9. Procédé d'obtention d'une fraction cellulaire possédant des propriétés anti-microbiennes et/ou immunomodulatrices, **caractérisé en ce que** ladite fraction est obtenue à partir d'une culture d'une souche de *Lactobacillus rhamnosus* selon une quelconque des revendications 1 ou 3.

10. Procédé selon la revendication 9, **caractérisé en ce que** ladite fraction cellulaire est choisie parmi :

      - une préparation d'ADN ou une préparation de parois bactériennes ;
      - un surnageant de culture, ou une fraction dudit surnageant.

11. Procédé d'obtention d'une composition possédant des propriétés anti-microbiennes et/ou immunomodulatrices, **caractérisé en ce qu'**il comprend l'obtention d'une fraction cellulaire par un procédé selon une quelconque des revendications 9 ou 10, et l'incorporation de ladite fraction dans ladite composition.

12. Procédé selon la revendications 11, **caractérisé en ce que** ladite composition est un produit alimentaire.

**Patentansprüche**

1. *Lactobacillus rhamnosus-Stamm,* der antimikrobielle Eigenschaften, immunmodulierende Eigenschaften und die Eigenschaft der spezifischen Haftung an Mannose aufweist, der bei der CNCM (COLLECTION NATIONALE DE CULTURES DE MICRO-ORGANISMES) unter der Nummer I-3690 hinterlegt ist.

2. Verfahren zur Gewinnung eines *Lactobacillus rhamnosus-Stammes,* **dadurch gekennzeichnet, dass** es die Mutagenese oder die genetische Transformation des Stammes CNCM I-3690 umfasst.

3. *Lactobacillus rhamnosus-Stamm,* **dadurch gekennzeichnet, dass** er nach dem Verfahren gemäß Anspruch 2 erhältlich ist und, dass er die antimikrobiellen Eigenschaften und die immunmodulierenden Eigenschaften des Stammes CNCM I-3690 aufweist.

4. Zusammensetzung enthaltend einen *Lactobacillus rhamnosus-Stamm* gemäß irgendeinem der Ansprüche 1 oder 3.

5. Zusammensetzung gemäß Anspruch 4, **dadurch gekennzeichnet, dass** es sich um ein Nahrungsmittelprodukt handelt.

6. *Lactobacillus rhamnosus-Stamm* gemäß irgendeinem der Ansprüche 1 oder 3 zur Verwendung als Arzneimittel.

7. *Lactobacillus rhamnosus-Stamm* zur Verwendung als Arzneimittel gemäß Anspruch 6, **dadurch gekennzeichnet, dass** das Arzneimittel zur Behandlung einer mikrobiellen Infektion bestimmt ist.

8. *Lactobacillus rhamnosus-Stamm* zur Verwendung als Arzneimittel gemäß Anspruch 6, **dadurch gekennzeichnet, dass** das Arzneimittel entzündungshemmend ist.

9. Verfahren zur Gewinnung einer Zellfraktion, die antimikrobielle Eigenschaften und/oder immunmodulierende Eigenschaften aufweist, **dadurch gekennzeichnet, dass** die Fraktion aus einer Kultur eines *Lactobacillus rhamnosus-Stammes* gemäß irgendeinem der Ansprüche 1 oder 3 erhalten wird.

10. Verfahren gemäß Anspruch 9, **dadurch gekennzeichnet, dass** die Zellfraktion ausgewählt ist aus:

    - einer DNA-Präparation oder einer Bakterienzellwand-Präparation;
    - einem Kulturüberstand oder einer Fraktion dieses Überstandes.

11. Verfahren zur Gewinnung einer Zusammensetzung, die antimikrobielle Eigenschaften und/oder immunmodulierende Eigenschaften aufweist, **dadurch gekennzeichnet, dass** es die Gewinnung einer Zellfraktion mit einem Verfahren gemäß irgendeinem der Ansprüche 9 oder 10 und die Beimischung dieser Fraktion zu der Zusammensetzung umfasst.

12. Verfahren gemäß Anspruch 11, **dadurch gekennzeichnet, dass** die Zusammensetzung ein Nahrungsmittelprodukt ist.

**Claims**

1. A *Lactobacillus rhamnosus* strain which has antimicrobial, immunomodulatory and mannose-specific adhesion properties, deposited with the CNCM under number I-3690.

2. A method for obtaining a *Lactobacillus rhamnosus* strain, **characterized in that** it comprises mutagenesis or genetic transformation of the CNCM I-3690 strain.

3. A *Lactobacillus rhamnosus* strain, **characterized in that** it can be obtained by means of the method as claimed in claim 2, and **in that** it has the antimicrobial properties and the immunomodulatory properties of the CNCM I-3690 strain.

4. A composition comprising a *Lactobacillus rhamnosus* strain as claimed in either one of claims 1 and 3.

**5.** The composition as claimed in claim 4, **characterized in that** it is a food product.

**6.** The *Lactobacillus rhamnosus* strain as claimed in either one of claims 1 and 3, for use as a medicament.

**7.** The *Lactobacillus rhamnosus* strain for use as a medicament as claimed in claim 6, **characterized in that** said medicament is intended for the treatment of a microbial infection.

**8.** The *Lactobacillus rhamnosus* strain for use as a medicament as claimed in claim 6, **characterized in that** said medicament is anti-inflammatory.

**9.** A method for obtaining a cell fraction which has antimicrobial and/or immunomodulatory properties, **characterized in that** said fraction is obtained from a culture of a *Lactobacillus rhamnosus* strain as claimed in either one of claims 1 and 3.

**10.** The method as claimed in claim 9, **characterized in that** said cell fraction is chosen from:

- a DNA preparation or a bacterial wall preparation;
- a culture supernatant, or a fraction of said supernatant.

**11.** A method for obtaining a composition which has antimicrobial and/or immunomodulatory properties, **characterized in that** it comprises obtaining a cell fraction by means of a method as claimed in either one of claims 9 and 10, and incorporating said fraction into said composition.

**12.** The method as claimed in claim 11, **characterized in that** said composition is a food composition.

Figure 1

CINAC I-3690 sur lait écrémé reconstitué

Legend:
— I-3690 ensemencement 5,5 106 UFC /mL
— I-3690 ensemencement 1,1 107 UFC /mL
— I-3690 ensemencement 2,2 107 UFC /mL
— I-3690 ensemencement 3,3 107 UFC /mL

pH axis: 6,3 6,1 5,9 5,7 5,5 5,3 5,1 4,9 4,7 4,5 4,3 4,1 3,9 3,7 3,5

Durée (h): 0 2 4 6 8 10 12 14 16 18 20 22 24 26 28 30 32 34 36 38 40 42 44 46

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2004093898 A
- EP 0817640 A
- EP 0577903 A
- WO 2004032639 A
- US 4839281 A
- WO 9910476 A
- WO 9391823 A
- WO 2004034808 A
- EP 1189517 A

**Littérature non-brevet citée dans la description**

- *Rapport FAO/OMS sur l'évaluation des propriétés sanitaires et nutritionnelles des probiotiques dans les aliments, y compris le lait en poudre contenant des bactéries lactiques vivants,* 01 Octobre 2001
- *Collection Nationale de Cultures de Microorganismes,* 09 Novembre 2006
- **MICHAIL ; ABERNATHY.** *Pediatr. Gastroenterol. Nutr.,* 2002, vol. 35, 350-355
- **MANGELL et al.** *Dig Dis Sci.,* 2002, vol. 47, 511-506
- **GURY et al.** *Arch Microbiol.,* 2004, vol. 182, 337-45
- **VÉLEZ et al.** *Appl Environ Microbiol.,* 2007, vol. 73, 3595-3604
- **PATNAIK et al.** *Nat Biotechnol,* 2002, vol. 20, 707-12
- **WANG Y. et al.** *J Biotechnol.,* 2007, vol. 129, 510-15
- **TERZAGHI ; SANDINE.** *Appl. Microbiol.,* 1975, vol. 29, 807-813
- **ELLIKER et al.** *J. Dairy Sci.,* 1956, vol. 39, 1611-1612
- **DE MAN et al.** *J. Appl. Bacteriol.,* 1960, vol. 23, 130-135
- **TIEN et al.** *J. Immunol.,* 2006, vol. 176, 1228-37